(19) Europäisches Patentamt
European Patent Office
Office européen des brevets

(11) Veröffentlichungsnummer: **0 069 288**
**B1**

# (12) EUROPÄISCHE PATENTSCHRIFT

(45) Veröffentlichungstag der Patentschrift: **08.05.85**

(51) Int. Cl.[4]: **C 07 D 213/75**, C 07 D 213/89, A 01 N 47/36

(21) Anmeldenummer: **82105595.1**

(22) Anmeldetag: **25.06.82**

## (54) N-Benzoyl-N'-pyridylharnstoffe, Verfahren zu ihrer Herstellung und ihre Verwendung zur Bekämpfung von Schädlingen.

(30) Priorität: **03.07.81 DE 3126263**

(43) Veröffentlichungstag der Anmeldung: **12.01.83 Patentblatt 83/2**

(45) Bekanntmachung des Hinweises auf die Patenterteilung: **08.05.85 Patentblatt 85/19**

(84) Benannte Vertragsstaaten: **AT BE CH DE FR GB IT LI NL SE**

(56) Entgegenhaltungen: **EP - A - 0 008 881**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Lange, Arno, Dr., Friedrichsplatz 11, D-6800 Mannheim (DE)**
Erfinder: **Parg, Adolf, Dr., Paray-le-Monial-Strasse 8, D-6702 Bad Duerkheim (DE)**
Erfinder: **Kiehs, Karl, Dr., Sudetenstrasse 22, D-6840 Lampertheim (DE)**
Erfinder: **Becker, Rainer, Dr., Sonnenwendstrasse 83, D-6702 Bad Duerkheim (DE)**
Erfinder: **Adolphi, Heinrich, Dr., Kalmitweg 11, D-6703 Limburgerhof (DE)**

EP 0 069 288 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft N-Benzoyl-N'-pyridylharnstoffe, Verfahren zu ihrer Herstellung und Schädlingsbekämpfungsmittel, die diese Verbindungen als Wirkstoffe enthalten.

Es ist bereits bekannt, daß N-Benzoyl-N'-pyridylharnstoffe als Insektizide verwendet werden können (EP-OS 8 881).

Es wurde nun gefunden, daß N-Benzoyl-N'-pyridylharnstoffe der Formel

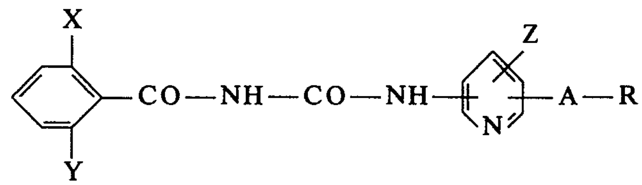
(I)

in der

X für Chlor, Fluor oder Wasserstoff und
Y für Chlor oder Fluor stehen,
mit der Maßgabe, daß X und Y nicht gleichzeitig Chlor bedeuten, und
Z für Wasserstoff oder Chlor und R für 4-Chlorphenyl, 2,4-Dichlorphenyl oder 3-Trifluormethylphenyl stehen, wenn A Sauerstoff bedeutet, oder
Z für Wasserstoff und R für 4-Chlorphenyl stehen und der Pyridinring in 2-Stellung durch den Rest

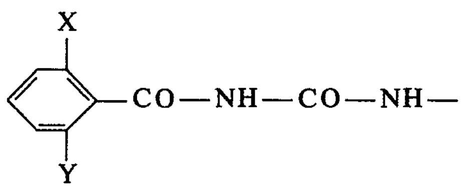

und in 5-Stellung durch den Rest —AR substituiert ist, wenn A Schwefel bedeutet, und N-Oxide der Verbindungen, bei denen A Sauerstoff bedeutet,

Schädlinge, insbesondere Insekten, wirksamer bekämpfen als bekannte N-Benzoyl-N'-pyridylharnstoffe.

Bevorzugte Verbindungen der Formel I sind solche, bei denen X und Y für Fluor, der Rest —AR in para-Stellung zum Rest

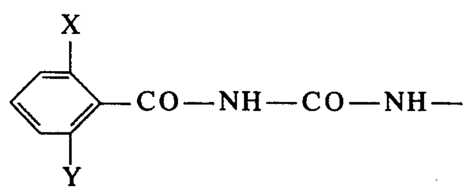

stehen.

Man erhält die N-Benzoyl-N'-pyridylharnstoffe durch Umsetzung

a) einer Verbindung der Formel

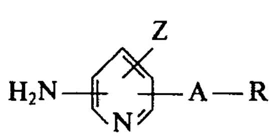
(II)

in der Z und R die obengenannten Bedeutungen haben, oder eines N-Oxids dieser Verbindung mit einem Benzoylisocyanat der Formel

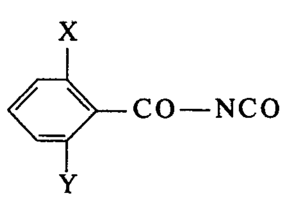
(III)

in der X und Y die obengenannten Bedeutungen haben, oder

b) einer Verbindung der Formel

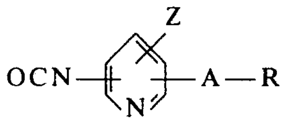

(IV)

in der Z und R die obengenannten Bedeutungen haben, oder eines N-Oxids dieser Verbindung mit einem Benzamid der Formel

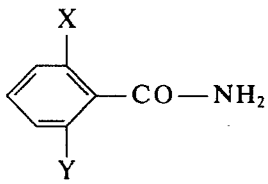

(V)

in der X und Y die obengenannten Bedeutungen haben.

Beide Umsetzungen können gegebenenfalls in Lösungs- bzw. Verdünnungsmitteln durchgeführt werden. Als solche kommen praktisch alle inerten organischen Solventien in Betracht. Hierzu gehören insbesondere aliphatische und aromatische, gegebenenfalls chlorierte oder nitrierte Kohlenwasserstoffe, wie Benzol, Toluol, Xylole, Chlorbenzole, Benzin, Tetrachlorkohlenstoff, 1,2-Dichlorethan, Methylenchlorid, Chloroform, Nitromethan, cyclische und acyclische Ether, wie Diethylether, Dibutylether, Tetrahydrofuran, Dioxan, acyclische und cyclische Ketone, wie Aceton, Methylethylketon, Methylisopropylketon, Cyclohexanon, außerdem Nitrile, wie Acetonitril, Benzonitril. Auch Gemische dieser Lösungsmittel können verwendet werden.

Bei der Umsetzung a) kann die Reaktionstemperatur innerhalb eines größeren Bereiches variiert werden. Sie liegt in der Regel zwischen 0 und 80°C,vorzugsweise zwischen 20 und 60°C. In diesem bevorzugten Bereich verläuft die Umsetzung ohne zusätzliche Kühlung. Bei der Umsetzung b) kann die Temperatur zwischen 0 und 140°C variiert werden, vorzugsweise arbeitet man bei einer Temperatur zwischen 80 und 130°C.

Man läßt die Umsetzung im allgemeinen bei Normaldruck ablaufen.

Die Reaktionskomponenten werden vorzugsweise in äquimolarem Verhältnis eingesetzt. Ein Überschuß der einen oder anderen Komponente bringt keine wesentlichen Vorteile. Die Umsetzung verläuft praktisch quantitativ. Es kann auch ein Katalysator, beispielsweise Triethylamin, zugesetzt werden.

Die erfindungsgemäßen Verbindungen fallen in der Regel analysenrein an; andernfalls können sie durch Umkristallisieren gereinigt werden. Zu ihrer Charakterisierung dienen Elementaranalyse und Schmelzpunkt.

Zur Durchführung der Verfahrensvariante a) wird zweckmäßigerweise das substituierte Aminopyridin der Formel II zusammen mit dem Lösungs- oder Verdünnungsmittel vorgelegt, dann wird eine äquimolare Menge an Isocyanat der Formel III zugegeben. Nach mehrstündiger Reaktionsdauer, in der Regel nach zwei Stunden, wird das Produkt dann abgesaugt, oder die Reaktionslösung wird am Rotationsverdampfer vom Lösungsmittel befreit. Anschließend wird das Produkt unter vermindertem Druck getrocknet. Bei der Verfahrensvariante b) beträgt die Reaktionsdauer 2 bis 6 Stunden.

Die Aminopyridine der Formel II, die Benzoylisocyanate der Formel III, die Pyridylisocyanate der Formel IV und die Benzamide der Formel V lassen sich nach bekannten Methoden herstellen (J. Het. Chem. 7, 1219–1222 (1970), J. Org. Chem. 28, 1805–1811 (1963)). Die Aminogruppe der Verbindungen der Formel II kann nach übichen Verfahren, z. B. durch Umsetzung mit Phosgen, in die Isocyanatgruppe umgewandelt werden, wobei die Verbindungen der Formel IV erhalten werden.

Die N-Oxidverbindungen der Formel

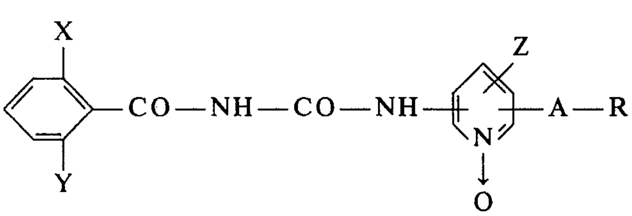

können auch durch Umsetzung der N-Benzoyl-N'-pyridylharnstoffe mit anorganischen oder organischen Oxidationsmitteln in Gegenwart eines inerten organischen Lösungsmittels erhalten werden. Vorzugsweise arbeitet man mit Wasserstoffperoxid in Eisessig oder 3-Chlorperbenzoesäure in Methylenchlorid.

Herstellungsbeispiele

1. 4,64 g 5-Amino-2-(4-chlorphenoxy)-pyridin werden in 80 ml Toluol vorgelegt. Dann tropft man 3,84 g 2,6-Difluorbenzoylisocyanat in 5 ml Toluol zu. Die Temperatur steigt um 26°C auf 39°C. Man hält eine Stunde bei 50°C und saugt bei Raumtemperatur ab. Man erhält 7,1 g N-2,6-Difluorbenzoyl-N'-[5-(4-chlorphenoxy)-pyrid-3-yl]-harnstoff vom Fp. 218–220°C.
2. 10 g des nach Beispiel 1 hergestellten Harnstoffs werden in 100 ml Chloroform suspendiert und mit 10 g 3-Chlorperbenzoesäure versetzt. Dann hält man 4 Stunden bei 40°C. Die Benzoesäure wird mit Natriumhydrogencarbonatlösung ausgewaschen. Die Chloroformsuspension wird abgesaugt, und der Rückstand wird getrocknet. Man erhält 7,2 g N-2,6-Difluorbenzoyl-N'-[5-(4-chlorphenoxy)-N''-oxipyrid-3-yl]-harnstoff, der nach dem Umkristallisieren aus Toluol-Aceton bei 173 bis 175°C schmilzt.

Analog werden folgende Harnstoffe und deren N-Oxide hergestellt:

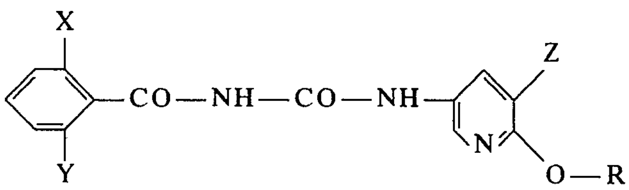

| Nr. | Y | X | Z | R | Fp [°C] |
|---|---|---|---|---|---|
| 3 | Cl | H | H | 4-Chlorphenyl | 185–187 |
| 4 | F | H | H | 4-Chlorphenyl | 173–176 |
| 5 | F | H | H | 2,4-Dichlorphenyl | 190–192 |
| 6 | Cl | H | H | 2,4-Dichlorphenyl | 182–185 |
| 7 | F | F | H | 2,4-Dichlorphenyl | 206–210 |
| 8 | F | F | Cl | 4-Chlorphenyl | 197–198 |
| 9 | Cl | H | Cl | 2,4-Dichlorphenyl | 184–186 |
| 10 | F | F | Cl | 2,4-Dichlorphenyl | 203–205 |

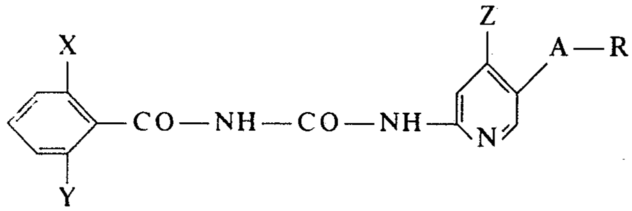

| Nr. | Y | X | Z | A | R | Fp [°C] |
|---|---|---|---|---|---|---|
| 11 | F | F | H | O | 2,4-Dichlorphenyl | 174–176 |
| 12 | Cl | H | H | O | 2,4-Dichlorphenyl | |
| 13 | F | F | H | O | 3-Trifluorphenyl | 186–188 |
| 14 | Cl | H | H | O | 3-Trifluorphenyl | |
| 15 | N-Oxid der Verbindung 12 | | | | | 78– 83 |
| 16 | F | F | H | S | 4-Chlorphenyl | 164–167 |
| 17 | N-Oxid der Verbindung 13 | | | | | 181–183 |

Die N-Benzoyl-N'-pyridylharnstoffe der Formel I sind geeignet, Schädlinge aus der Klasse der Insekten und Spinnentiere wirksam zu bekämpfen. Sie sind geeignete Adultizide und Ovizide und können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria bioliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer) Galleria mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceutorrhynchus napi (Großer Kohltriebrüßler), Sitophilus granaria (Kornkäfer), Anisandrus dispar (Ungleicher Holzborkenkäfer), Ips typographus (Buchdrucker),

Blastophagus piniperda (Gefurchter Waldgärtner);

aus der Ordnung der Zweiflügler (Diptera) beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipuola oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Subenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypodermia lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispiesweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Zur Klasse der Arachnoidea gehören Spinnentiere (Acarina), beispielsweise Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feine Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 3 Gewichtsteile der Verbindung Nr. 1 werden mit 97 Gewichtsteilen feinteiligem Kaolin innig vermischt. Man erhält auf diese Weise ein Stäubemittel, das 3 Gew.-% des Wirkstoffs enthält.

II. 30 Gewichtsteile der Verbindung Nr. 10 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 11 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 5 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 13 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibrom-ethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methyl-carbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methyl-carbamoyl)-oxim, S-Methyl-N-[(methylcarbamoyl)-oxy]-thio-acetimidat, Methyl-N,N'-dimethyl-N-[(methylcarbamoyl)oxy]-1-thiooxamidat, N-(2-Methyl-chlor-phenyl)-N,N'-dimethylformamidin, Tetrachlorthiophen, 1-(2,6-Difluor-benzoyl)-3-(4-chlor-phenyl)-harnstoff, O,O-Dimethyl-O-(p-nitrophenyl)-phosphorthioat, O,O-Diethyl-O-(p-nitrophenyl)-phosphorthioat, O-Ethyl-O-(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphonothioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-[p-methylsulfinyl-phenyl]-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[-2-chlor-1-(2,4,5-trichlorphenyl)]-vinyl-phosphat, O,O-Dimethyl-S-(1'-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methyl-carbamoyl-methyl)-phosphorthioat, O,O-Dimethyl-S-(N-methoxyethyl-carbamoyl-methyl)-phosphordithioat, O,O-Dimethyl-S-(N-formyl-N-methyl-carbamoylmethyl-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethyl-thio-methyl)-phosphordithioat, O,O-Diethyl-S-[(p-chlor-phenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinyl-ethyl)-phosphorthioat, O,O-Diethyl-thiophosphoryliminophenyl-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]phosphorthioat, O,O-Diethyl-O-(2-pyrazinyl)-phosphorthioat, O,O-Diethyl-O-(2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphoramido-thioat, O,S-Dimethyl-N-acetyl-phosphoramidothioat, γ-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on(1)-yl-(4)-DL-cis,trans-chrysanthemat, 5-Benzyl-furyl-(3)-methyl-DL-cis,trans-chrysanthemat, 3-Phenoxybenzyl(±)-cis,trans-2,2-dimethyl-3-(2,2-dichlorvinyl)-cyclopropancarboxylat, α-Cyano-3-phenoxybenzyl(±)-cis,trans-2,2-dimethyl-3-(2,2-dichlorvinyl)-cyclopropancarboxylat, (s)-α-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,trans-chrysanthemat, 2-Methyl-5-(2-propinyl)-3-furylmethyl-chrysanthemat, (α-Cyano-3-phenoxybenzyl)-α-isopropyl-4-chlorphenylacetat.

Die folgenden Beispiele belegen die biologische Wirkung der neuen Verbindungen. Vergleichsmittel sind die aus der EP-OS 8 881 bekannten Wirkstoffe N-(2,6-Dichlorbenzoyl)-N'-[2-(4-chlorphenoxy)-pyrid-5-yl]-harnstoff (A) und N-(2,6-Difluorbenzoyl)-N'-[2-(4-chlorphenylthio)-pyrid-5-yl]-harnstoff (B).

Die Numerierung der Wirkstoffe entspricht der der tabellarischen Auflistung.

## Beispiel 1

### Zuchtversuch mit Moskito-Larven (Aedes aegypti)

200 ml Leitungswasser werden mit der Wirkstoffaufbereitung, die 10 Gew.-% Wirkstoff und 90 Gew.-% eines Emulgatorgemisches aus 10 Gew.-% ethoxyliertem Rizinusöl, 20 Gew.-% ethoxyliertem Isooctylphenol und 70 Gew.-% Cyclohexanon enthält, versetzt und darauf mit 30 bis 40 Aedes-Larven im 4 Stadium belegt.

Die Versuchstemperatur beträgt 25°C. Beurteilt werden Verpuppung und Schlüpfen der Imagines, wobei eine unbehandelte Kontrolle als Maßstab dient. Während der Versuchsdauer von 10 bis 12 Tagen wird einmal mit einem herkömmlichen pulverisierten Fischfutter gefüttert.

In diesem Test zeigen die Wirkstoffe Nr. 7, 8, 10, 11, 16 eine bessere Wirkung als die Vergleichsmittel.

## Beispiel 2

Zuchtversuch mit Maiszünsler-Larven (Ostrinia nubilalis)

Die Zucht erfolgt auf einem Nährboden folgender Zusammensetzung:

| | |
|---|---|
| 515 g | Maismehl |
| 130 g | Weizenkeime |
| 137 g | Bierhefe |
| 18 g | Ascorbinsäure |
| 10 g | Cellulosepulver |
| 5 g | p-Hydroxibenzoesäuremethylester |
| 20 g | Spurenelementmischung nach Wesson |
| 20 ml | Vitaminlösung |
| 80 g | Agar |
| 3100 ml | Wasser |

Je 50 ml des Nährbodens füllt man in 100-ml-Plastikbecher und mischt die wäßrige Wirkstoffaufbereitung sorgfältig unter.

Nach dem Erkalten belegt man jedes Gefäß mit 4 Raupen (L 3). Pro Konzentration werden 5 Gefäße angesetzt. Die Beobachtung erstreckt sich bis zum Schlüpfen der Falter.

In diesem Test zeigen die Wirkstoffe Nr. 1, 11, 16 eine bessere Wirkung als das Vergleichsmittel A.

## Beispiel 3

Wirkung auf Eiablagen der Gewächshausspinnmilbe
(Tetranychus telarius)

Bohnenpflanzen werden in der Spritzkabine mit den Wirkstoffaufbereitungen tropfnaß gespritzt. Aus diesen Blättern stanzt man Stücke von 25 mm Durchmesser. Diese Blattstücke werden auf ein Zellstoffstück gelegt, dessen Seiten von Wasser benetzt werden. Jedes Stanzstück belegt man mit 5 adulten Weibchen der Gewächshausspinnmilbe. Nach 12 Tagen erfolgt die Auswertung auf abgelegte, schlüpffähige Eier bzw. geschlüpfte Larven.

In diesem Test zeigt der Wirkstoff Nr. 11 eine bessere Wirkung als das Vergleichsmittel A.

**Patentansprüche für die Vertragsstaaten BE, CH, DE, FR, GB, IT, LI, NL, SE**

1. N-Benzoyl-N'-pyridylharnstoffe der Formel

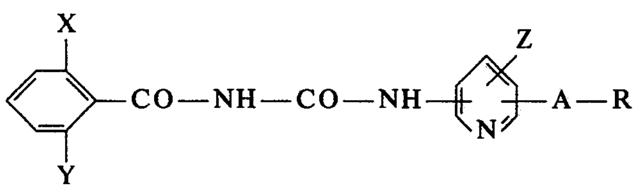

(I)

in der

X für Chlor, Fluor oder Wasserstoff und
Y für Chlor oder Fluor stehen,
mit der Maßgabe, daß X und Y nicht gleichzeitig Chlor bedeuten, und
Z für Wasserstoff oder Chlor und R für 4-Chlorphenyl, 2,4-Dichlorphenyl oder 3-Trifluormethylphenyl stehen, wenn A Sauerstoff bedeutet, oder
Z für Wasserstoff und R für 4-Chlorphenyl stehen und der Pyridinring in 2-Stellung durch den Rest

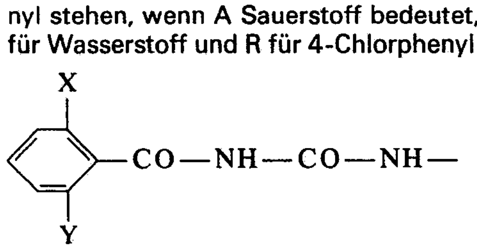

und in 5-Stellung durch den Rest —AR substituiert ist, wenn A Schwefel bedeutet, und N-Oxide der Verbindungen, bei denen A Sauerstoff bedeutet.

2. N-Benzoyl-N'-pyridylharnstoffe der Formel

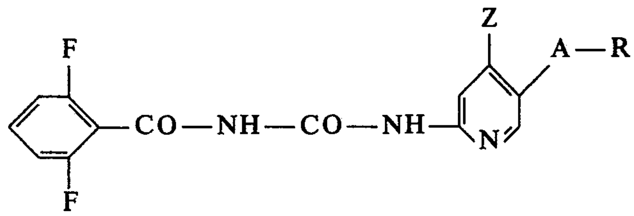

in der
Z, A und R die im Anspruch 1 genannten Bedeutungen haben.

3. N-Benzoyl-N'-pyridylharnstoffe der Formel

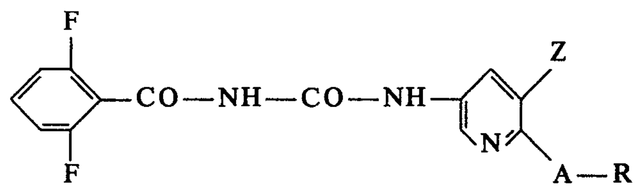

in der
Z, A und R die im Anspruch 1 genannten Bedeutungen haben.

4. Verfahren zur Herstellung von N-Benzoyl-N'-pyridylharnstoffen der Formel I gemäß Anspruch 1, dadurch gekennzeichnet, daß man

a) einer Verbindung der Formel

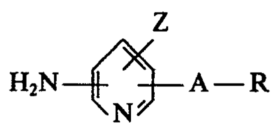 (II)

in der Z und R die im Anspruch 1 genannten Bedeutungen haben, oder ein N-Oxid dieser Verbindung mit einem Benzoylisocyanat der Formel

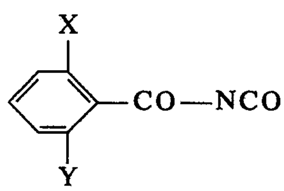 (III)

in der X und Y die im Anspruch 1 genannten Bedeutungen haben, oder

b) einer Verbindung der Formel

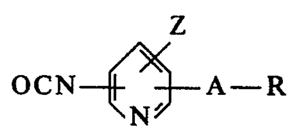 (IV)

in der Z und R die im Anspruch 1 genannten Bedeutungen haben, oder ein N-Oxid dieser Verbindung mit einem Benzamid der Formel

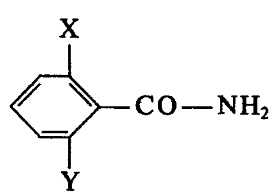 (V)

in der X und Y die obengenannten Bedeutungen haben.

5. Schädlingsbekämpfungsmittel, enthaltend einen N-Benzoyl-N'-pyridylharnstoff der Formel I gemäß Anspruch 1.

6. Schädlingsbekämpfungsmittel, enthaltend inerte Zusatzstoffe und einen N-Benzoyl-N'-pyridylharnstoff der Formel I gemäß Anspruch 1.

7. Insektizides Mittel, enthaltend inerte Zusatzstoffe und einen N-Benzoyl-N'-pyridylharnstoff der Formel I gemäß Anspruch 1.

8. Verfahren zur Bekämpfung von Schädlingen, dadurch gekennzeichnet, daß man eine wirksame Menge eines N-Benzoyl-N'-pyridylharnstoffs der Formel I gemäß Anspruch 1 auf die Schädlinge und/oder deren Lebensraum einwirken läßt.

**Patentansprüche für den Vertragsstaat AT**

1. Schädlingsbekämpfungsmittel, enthaltend inerte Zusatzstoffe und einen N-Benzoyl-N'-pyridylharnstoff der Formel

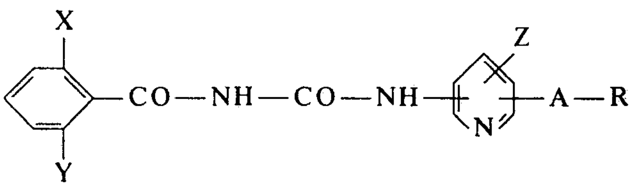

(I)

in der

X für Chlor, Fluor oder Wasserstoff und
Y für Chlor oder Fluor stehen,
mit der Maßgabe, daß X und Y nicht gleichzeitig Chlor bedeuten, und
Z für Wasserstoff oder Chlor und R für 4-Chlorphenyl, 2,4-Dichlorphenyl oder 3-Trifluormethylphenyl stehen, wenn A Sauerstoff bedeutet, oder
Z für Wasserstoff und R für 4-Chlorphenyl stehen und der Pyridinring in 2-Stellung durch den Rest

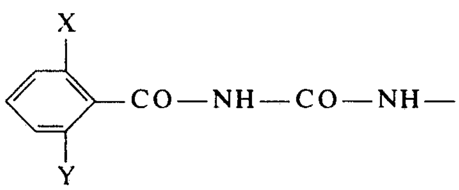

und in 5-Stellung durch den Rest —AR substituiert ist, wenn A Schwefel bedeutet,

oder N-Oxide der Verbindungen, bei denen A Sauerstoff bedeutet.

2. Schädlingsbekämpfungsmittel, enthaltend inerte Zusatzstoffe und einen N-Benzoyl-N'-pyridylharnstoff der Formel

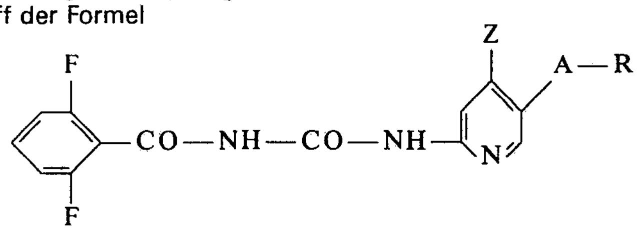

in der
Z, A und R die im Anspruch 1 genannten Bedeutungen haben.

3. Schädlingsbekämpfungsmittel, enthaltend inerte Zusatzstoffe und einen N-Benzoyl-N'-pyridylharnstoff der Formel

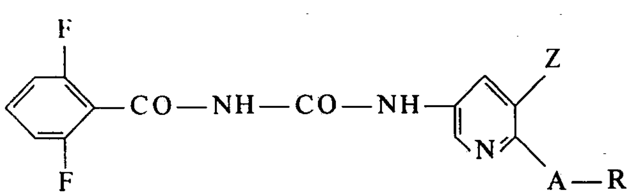

in der
Z, A und R die im Anspruch 1 genannten Bedeutungen haben.

4. Verfahren zur Herstellung von N-Benzoyl-N'-pyridylharnstoffen der Formel I gemäß Anspruch 1, dadurch gekennzeichnet, daß man

a) eine Verbindung der Formel

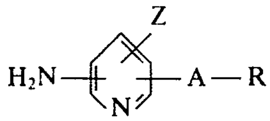 (II)

in der Z und R die im Anspruch 1 genannten Bedeutungen haben, oder ein N-Oxid dieser Verbindung mit einem Benzoylisocyanat der Formel

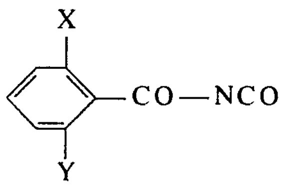 (III)

in der X und Y die im Anspruch 1 genannten Bedeutungen haben, oder

b) einer Verbindung der Formel

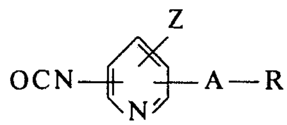 (IV)

in der Z und R die im Anspruch 1 genannten Bedeutungen haben, oder ein N-Oxid dieser Verbindung mit einem Benzamid der Formel

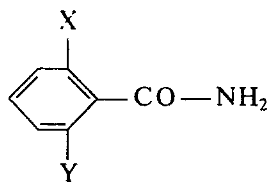 (V)

in der X und Y die im Anspruch 1 genannten Bedeutungen haben, umsetzt.

**Claims for the Contracting States BE, CH, DE, FR, GB, IT, LI, NL, SE**

1. N-benzoyl-N'-pyridyl ureas of the formula

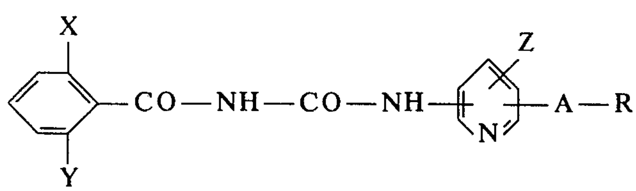 (I)

where

X is chlorine, fluorine or hydrogen, and

Y is chlorine or fluorine, with the proviso that X and Y do not simultaneously denote chlorine, and

Z is hydrogen or chlorine and R is 4-chlorophenyl, 2,4-dichlorophenyl or 3-trifluoromethyl when A is oxygen, or

Z is hydrogen and R is 4-chlorophenyl and the pyridine ring is substituted in the 2-position by the radical

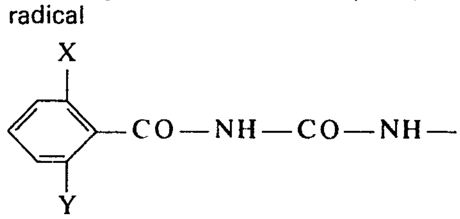

and in the 5-position by the radical —AR when A is sulphur,

and N-oxides of the compounds in which A denotes oxygen

2. N-benzoyl-N'-pyridyl ureas of the formula

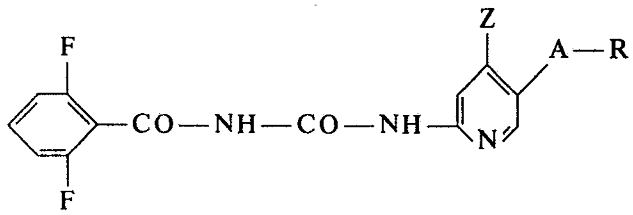

where Z, A and R have the meanings given in claim 1.

3. N-benzoyl-N'-pyridyl ureas of the formula

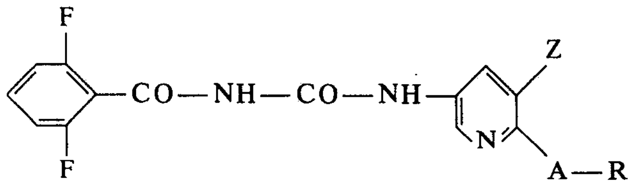

where Z, A and R have the meanings given in claim 1.

4. A process for the preparation of N-benzoyl-N'-pyridyl ureas of the formula I as claimed in claim 1, wherein

(a) a compound of the formula

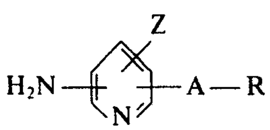 (II)

where Z and R have the meanings given in claim 1, or an N-oxide of this compound is reacted with a benzoyl isocyanate of the formula

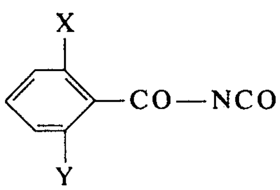 (III)

where X and Y have the meanings given in claim 1, or

b) a compound of the formula

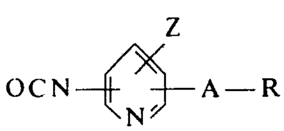 (IV)

where Z and R have the meanings given in claim 1, or an N-oxide of this compound is reacted with a benzamide of the formula

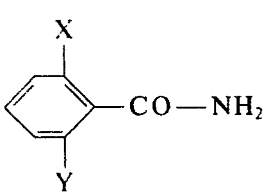 (V)

where X and Y have the meanings given in claim 1.

5. A pesticide containing an N-benzoyl-N'-pyridyl urea of the formula I as claimed in claim 1.

6. A pesticide containing inert carriers and an N-benzoyl-N'-pyridyl urea of the formula I as claimed in claim 1.

7. An insectical agent containing inert carriers and an N-benzoyl-N'-pyridyl urea of the formula I as

claimed in claim 1.

8. A method of combating pests, wherein an effective amount of an N-benzoyl-N'-pyridyl urea of the formula I as claimed in claim 1 is allowed to act on the pests and/or their habitat.

**Claims for the Contracting State AT**

1. A pesticide containing inert carriers and an N-benzoyl-N'-pyridyl urea of the formula

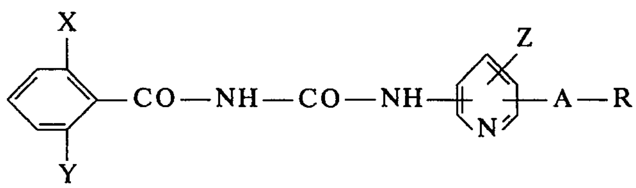

(I)

where

X is chlorine, fluorine or hydrogen, and
Y is chlorine or fluorine, with the proviso that X and Y do not simultaneously denote chlorine, and
Z is hydrogen or chlorine and R is 4-chlorophenyl, 2,4-dichlorophenyl or 3-trifluoromethyl when A is oxygen, or
Z is hydrogen and R is 4-chlorophenyl and the pyridine ring is substituted in the 2-position by the radical

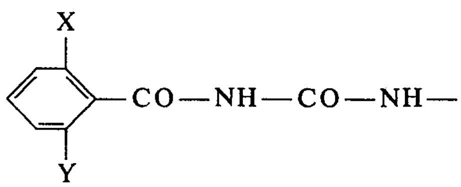

and in the 5-position by the radical —AR when A is sulphur,

or an N-oxide of the compound in which A denotes oxygen.

2. A pesticide containing inert carriers and an N-benzoyl-N'-pyridyl urea of the formula

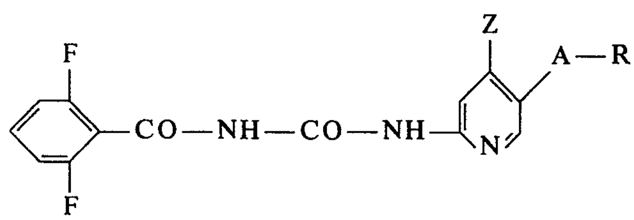

where Z, A and R have the meanings given in claim 1.

3. A pesticide containing inert carriers and an N-benzoyl-N'-pyridyl urea of the formula

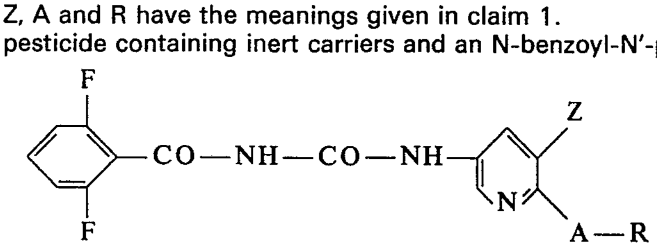

where Z, A and R have the meanings given in claim 1.

4. A process for the preparation of N-benzoyl-N'-pyridyl ureas of the formula I as defined in claim 1, wherein

(a) a compound of the formula

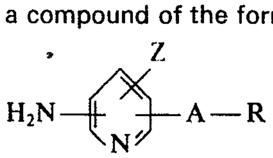

(II)

where Z and R have the meanings given in claim 1, or an N-oxide of this compound is reacted with a

benzoyl isocyanate ot fhe formula

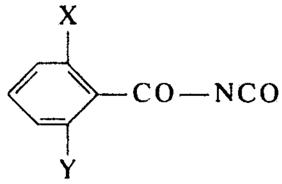

(III)

where X and Y have the meanings given in claim 1, or

b) a compound of the formula

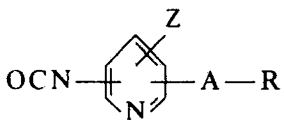

(IV)

where Z and R have the meanings given in claim 1, or an N-oxide of this compound is reacted with a benzamide of the formula

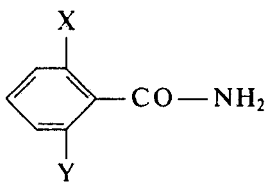

(V)

where X and Y have the meanings given in claim 1.

**Revendications pour les Etats contractants BE, CH, DE, FR, GB, IT, LI, NL, SE**

1. N-benzoyl-N'-pyridyl-urées de la formule

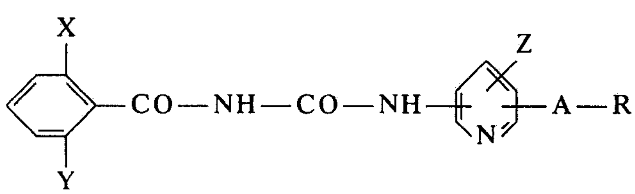

(I)

dans laquelle

A désigne un atome de chlore, de fluor ou d'hydrogène et
Y désigne un atome de chlore ou de fluor,
X et Y ne pouvant cependant pas désigner simultanément des atomes de chlore, et
Z désigne un atome d'hydrogène ou de chlore et
R un groupe chloro-4 phényle, dichloro-2,4 phényle ou trifluorométhyl-3 phényle, lorsque A représente un atome d'oxygène, ou
Z désigne un atome d'hydrogène et
R un groupe chloro-4 phényle et le noyau pyridine est substitué en position 2 par un groupe

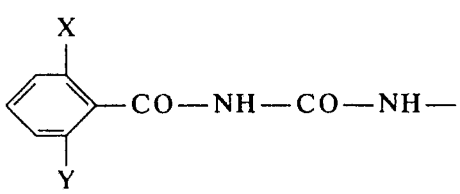

et en position 5 par un groupe —AR, lorsque A représente un atome de soufre,

et les N-oxydes des dérivés, pour lesquels A = O.

2. N-benzoyl-N'-pyridyl-urées de la formule

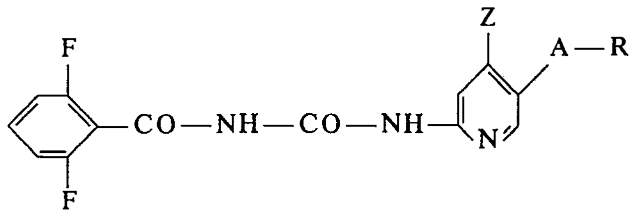

dans laquelle Z, A et R possédent les significations définies dans la revendication 1.

3. N-benzoyl-N'-pyridyl-urées de la formule

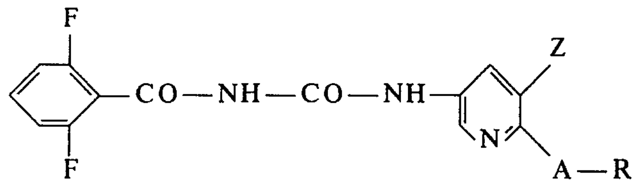

dans laquelle Z, A et R possèdent les significations définies dans la revendication 1.

4. Procédé de préparation de N-benzoyl-N'-pyridyl-urées de la formule I suivant la revendication 1, caractérisé en ce que l'on fait réagir:

a) un composé de la formule

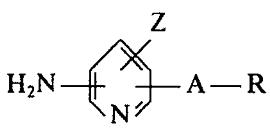

(II)

dans laquelle Z et R possèdent les significations définies dans la revendication 1, ou un N-oxyde d'un tel composé, avec un isocyanate de benzoyle de la formule

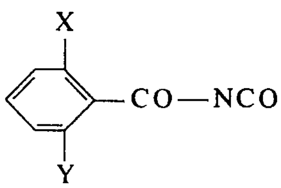

(III)

dans laquelle X et Y possèdent les significations définies dans la revendication 1, ou

b) un composé de la formule

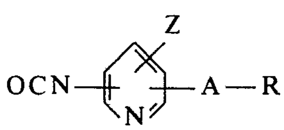

(IV)

dans laquelle Z et R possèdent les significations définies dans la revendication 1, ou un N-oxyde d'un tel composé, avec un benzamide de la formule

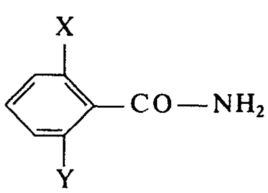

(V)

dans laquelle X et Y possèdent les significations définies dans la revendication 1.

5. Composition parasiticide, contenant une N-benzoyl-N'-pyridyl-urée de la formule I suivant la revendication 1.

6. Composition parasiticide, contenant une N-benzoyl-N'-pyridyl-urée de la formule I suivant la revendication 1, ainsi que des additifs inertes.

7. Composition insecticide, contenant une N-benzoyl-N'-pyridyl-urée de la formule I suivant la revendication 1, ainsi que des additifs inertes.

8. Procédé de lutte contre des parasites, caractérisé en ce que l'on fait agir une quantité efficace d'une N-benzoyl-N'-pyridyl-urée de la formule I suivant la revendication 1 sur les parasites et(ou) leur biotope.

**Revendications pour l'Etat contractant: AT**

1. Compositions parasiticides, contenant des additifs inertes et une N-benzoyl-N'-pyridyl-urée de la formule

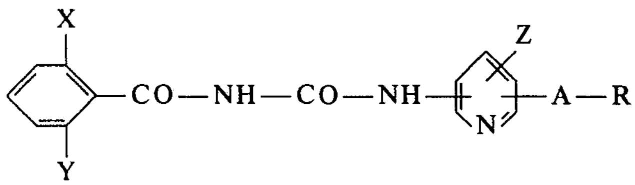

(I)

dans laquelle

X désigne un atome de chlore, de fluor ou d'hydrogène et
Y désigne un atome de chlore ou de fluor,
X et Y ne pouvant cependant pas désigner simultanément des atomes de chlore, et
Z désigne un atome d'hydrogène ou de chlore et
R un groupe chloro-4 phényle, dichloro-2,4 phényle ou trifluorométhyl-3 phényle, lorsque A représente un atome d'oxygène, ou
Z désigne un atome d'hydrogène et
R un groupe chloro-4 phényle et le noyau pyridine est substitué en position 2 par un groupe

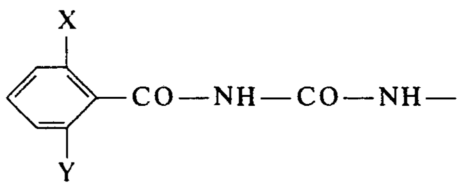

et en position 5 par un groupe —AR, lorsque A représente un atome de soufre,

ou des N-oxydes des dérivés, pour lesquels A = O.

2. Composition parasiticide, contenant des additifs inertes et une N-benzoyl-N'-pyridyl-urée de la formule

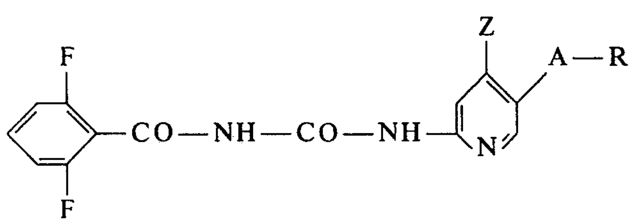

dans laquelle Z, A et R possèdent les significations définies dans la revendication 1.

3. Composition parasiticide, contenant des additifs inertes et une N-benzoyl-N'-pyridyl-urée de la formule

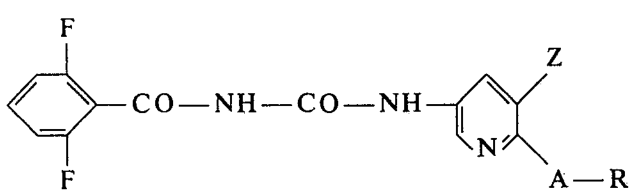

dans laquelle Z, A et R possèdent les significations définies dans la revendication 1.

4. Procédé de préparation de N-benzoyl-N'-pyridyl-urées de la formule I suivant la revendication 1, caractérisé en ce que l'on fait réagir:

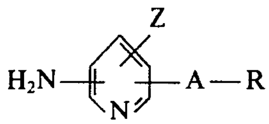 (II)

dans laquelle Z et R possèdent les significations définies dans la revendication 1, ou un N-oxyde d'un tel composé, avec un isocyanate de benzoyle de la formule

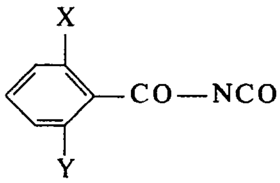 (III)

dans laquelle X et Y possèdent les significations définies dans la revendication 1, ou

b) un composé de la formule

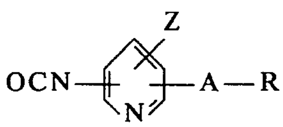 (IV)

dans laquelle Z et R possèdent les significations définies dans la revendication 1, ou un N-oxyde d'un tel composé, avec un benzamide de la formule

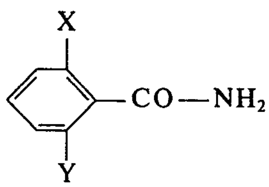 (V)

dans laquelle X et Y possèdent les significations définies dans la revendication 1.